Europäisches Patentamt

European Patent Office

Office européen des brevets

(11) Publication number: **0 012 774**
**B1**

(12) **EUROPEAN PATENT SPECIFICATION**

(45) Date of publication of patent specification: **05.09.84**

(51) Int. Cl.³: **A 61 K 35/44**

(21) Application number: **79900371.0**

(22) Date of filing: **19.03.79**

(86) International application number:
**PCT/JP79/00068**

(87) International publication number:
**WO 79/00854 01.11.79 Gazette 79/22**

(54) **VEGETATIVE STIGMATA THERAPEUTIC AGENT**

(30) Priority: **31.03.78 JP 38389/78**

(43) Date of publication of application:
**09.07.80 Bulletin 80/14**

(45) Publication of the grant of the patent:
**05.09.84 Bulletin 84/36**

(84) Designated Contracting States:
**CH DE FR GB**

(56) References cited:
FR-A-2 007 399
FR-A-2 138 509
FR-M- 7 347
GB-A-1 088 304
JP-B-29 003 698
US-A-2 583 096

DERWENT PUBLICATIONS; SOVIET INVENTIONS ILLUSTRATED, August 1964, page 4, ref. 160566, LONDON (GB) Z.N.

ZAPRUDSKAYA: "Regenerator preparation" CHEMICAL ABSTRACTS, vol. 80, 1974, abstract 142560h, page 148, COLUMBUS, OHIO (US)

(73) Proprietor: **SNOWDEN CO., LTD.**
**Marume Building 7-16, Iwamoto-cho 3-chome Chiyoda-ku Tokyo (JP)**

(72) Inventor: **KOSUGI, Kikuo**
**43-14, Kamitakada 1-chome Nakano-ku Tokyo 164 (JP)**
Inventor: **INUI, Susumu**
**6-22, Nishikubo 1-chome Shimizu-shi Shizuoka 424 (JP)**
Inventor: **INUI, Shigeru**
**6-22, Nishikubo 1-chome Shimizu-shi Shizuoka 424 (JP)**
Inventor: **ASUKABE, Yoshizo**
**30-27, Amanuma 2-chome Suginami-ku Tokyo 167 (JP)**

(74) Representative: **Rowe, Eric Nielsen et al**
**Edward Evans & Co. Chancery House 53-64 Chancery Lane London WC2A 1SD (GB)**

(56) References cited:
SHENG-SAN LEE: "Glycosaminoglycans in tissues. I. Method of extraction and preparation of acidic glycosaminoglycans from the human umbilical cord"

Courier Press, Leamington Spa, England.

# 0 012 774

**Description**

Technical Field

This invention relates to an autonomic imbalance treating agent prepared from human umbilical cords and which gives good results in clinical application.

Background Art

In recent years, many people have been suffering from autonomic imbalance, the susceptibility to which is influenced by complicated and diverse external factors such as domestic social or cultural problems. Such people are increasing in number. Autonomic imbalance is a condition that generally lacks symptoms of organic disease. In autonomic imbalance, by the dysfunction of autonomic nerves, the balance of the vegetative nervous system (sympathetic and para-sympathetic) is lost. Autonomic imbalance has equivocal symptoms such as ease of getting tired, stiff shoulders, headache, dizziness and benumbed extremities. Autonomic imbalance may be classified into three classes; neurotic autonomic imbalance, which is mainly of psychogenic origin; pysychosomatic autonomic imbalance, where equivocal symptoms are experienced according to the reciprocal interference of the psycho and autonomic central nervous system; and essential autonomic imbalance, which is caused by imbalance of the autonomic central nervous system. Climacteric disturbance of woman is also thought to be a symptom of autonomic imbalance. Autonomic imbalance is mainly experienced by people whose age in years is in the twenties and thirties and is experienced more by women than men. The occurrence of autonomic imbalance is said to be greater in summer and at changes of the seasons that at other times.

Autonomic imbalance is not only experienced by adults. Bronchial asthma, enuresis and cyclic vomiting in nursing babies and infants are said to be caused by autonomic imbalance. Pyschological situations such as anxiety and tension from their adolescent surroundings and constitutional abnormalities combined with present-day circumstances cause autonomic imbalance to occur among young people.

Autonomic imbalance exhibits for example the following symptoms caused by dysfunction of autonomic nerves: cyclic vomiting (autotoxication); nocturnal enuresis e.g. of nursing babies; autonomic imbalance of respiratory apparatus (constitutional iterative catarrhal symptoms); autonomic instability in children of primary school age; pyschogenic autonomic imbalance (anxiety, tension, suggestion somatisation), hypertensive intestinal syndromes, gastric neurosis and nervous vomiting in middle-, high-school and college students; autonomic lumbago (pelvic congestion); gynecological postoperative autonomic imbalance; postnatal and postaborative autonomic imbalance; autonomic vertigo in housewives; sensitiveness to cold in housewives; headache; dullhead; ease of getting tired; stiff shoulders; insomnia; gastrointestinal disturbance; chilly extremities; dizziness; aesthenopia; gastrointestinal neurosis; laryngeal neurosis; orthostatic disturbance; arthralgia; feeling of fatigue; frigidity; languor; feverish feeling; vertigo (in postcapital and cervical injury and otherwise); ear noise; fatigue; numbness of extremities; feeling of ease of getting tired; and cervical, lumbar, dorsal and scapular stiffness and pain.

The above symptoms may occur irrespective of age and sex except where stated or where peculiar to people of particular ages and/or sex. Some symptoms (headache, arthralgia, stiff shoulders, feeling of fatigue, frigidity, languor, feverish feeling, vertigo, ear noise, fatigue, chilly extremities, loss of appetite and numbness of extremities) may occur as a result of climacteric disturbances (climacteric autonomic nervous imbalance).

For the above-mentioned symptoms of autonomic imbalance, many kinds of treatments have been developed, for instance, autogenic training, simple psychosomatic therapy, transactional analysis, Morita's therapy, behaviour therapy, drug treatments and combinations of these therapies and other therapies. In drug treatment, depending on the patient's type, minor tranquilizers, anti-depressive agents, hypnotics, autonomic agents and others are skillfully administered and many of them are used combined with the aforesaid therapies. However, adverse reactions are caused by some of those drugs and some are insufficiently effective and hitherto there has not been a satisfactory safe and effective drug for the treatment of autonomic imbalance.

It is known from the following documents

(1) FR—A—2 138 509
(2) FR—M—7 347
(3) FR—A—2 007 399
(4) GB—A—1 088 304
(5) US—A—2 583 096
(6) Derwent Publications; Soviet Inventions illustrated, August 1964 page 4 reference 160566
(7) Chem. Abs. *80* (1974) 142560h

that products obtained by extracting human and animal umbilical cords have been found to have wide use in therapy: thus for example (1) describes a product for use in in immotherapy and (2) describes products useful for the treatment of vascular diseases. Methods are known for the extraction of various

2

products, particularly hyaluronic acid from umbilical cords. cf. (1), (2), (4), (5), (6) and (7). Physical breakdown of human umbilical cords is disclosed in e.g. (5) at example 1. Treatment of *inter alia* the protein from human umbilical cords by proteolysis is disclosed in (4) at lines 88—92 of page 2. The extraction of umbilical cords by solvents is disclosed in all of the documents (1), (2), (4), (5), (6) and (7). The desirability of removing proteins from solutions to be used for injection is well-known because of the discomfort and danger resulting from their presence, thus (6) refers in the second paragraph to the dangers involved in using unpurified hyaluronic acid extracted from human umbilical cords.

The inventors, after devoting themselves to research for a medicament for the treatment of autonomic imbalance, have developed a new autonomic imbalance treating agent prepared from human umbilical cords and giving good results in clinical application.

In accordance with the present invention, there is provided an autonomic imbalance treating agent prepared by treating comminuted human umbilical cords in an aqueous medium with a proteolytic enzyme to effect enzymic decomposition, removing proteins from the resulting solution and sterilizing the same free of proteins.

Details of this invention are described below.

The human umbilical cords utilized in the present invention should be fresh. However, if it is difficult to obtain a large quantity of fresh umbilical cords at one time on an industrial production scale, umbilical cords which have been frozen when fresh and then stored, may be used. The aforesaid frozen cords are washed, thawed and blood is removed with purified water or other liquid. They are then rinsed with cold water. The cords after removal of the blood can be used directly in the next step but alternatively may be kept cold or freeze-stored or freeze-dried for use later. The umbilical cords after removal of the blood and washing are cut into small pieces and then minced and crushed.

The aforesaid aqueous medium may be water, physiological saline, an aqueous alcohol solution or another aqueous solution. The medium is preferably water or physiological saline. The medium may contain a pH adjuster.

The umbilical cords may be subjected to, in addition to the enzymic decomposition, physical treatment such as ultrasonic treatment, mechanical grinding and repeated freezing and thawing.

The stroma of umbilical cords is histologically a connective glia tissue and principally contains collagen, protein and acid mucopolysaccharides, which are strongly combined with each other. The umbilical cord extract of this invention principally contains a full content of amino-nitrogen, mucopolysaccharides and acid mucopolysaccarides. According to the present invention, instead of mere physical decomposition of the cells of umbilical cords, proteins combined with mucopolysaccharides are decomposed and an extract is obtained containing a considerably larger amount of effective constituents compared with the extracts obtained by the solely physical treatment.

Protein decomposition, that is, proteolysis may be effected by acid decomposition using inorganic acids such as hydrochloric acid or sulphuric acid; or organic acids such as citric acid and by enzymolysis using proteolytic enzymes. However, according to the present invention, protein decomposition is effected by enzymolysis.

Comparing acid decomposition with enzymolysis, acid decomposition is difficult to control. For instance, if a strong acid is used there is a risk that mucopolysaccharides which are thought to be effective may be destroyed, and on the contrary there may be a shortcoming that if a weak acid is used, decomposition of the protein itself may not be completed. Enzyme decomposition of protein, that is, enzymolysis does not have these shortcomings and the decomposition is gentle and can be a better way of extraction.

The proteolytic enzymes that can be used in the invention include almost all known proteolytic enzyme. Such enzymes include enzymes of animal origin, such as pepsin, trypsin and chymotrypsin, enzymes originating from microorganisms such as *Streptomyces griseus, Bacillus subtilis, Aspergillus melius, Aspergillus niger*, and other bacteria such as *Serratia* or *Bacillus*, enzymes originating from plants such as bromelain, papain and facin, and fixed enzymes which may be obtained by modification of proteolytic enzymes by well known processes.

Suitable conditions for the decomposition using a proteolytic enzyme may differ depending on the prior treatment of the umbilical cords and/or the particular proteolytic enzymes used but suitable conditions of liquid pH, enzyme activity, reaction time and reaction temperature may be suitably selected.

Preferably the reaction temperature is 40—60°C and the reaction time is 4—20 hours. It is sufficient that almost no residual enzyme activity may be left after a given time of reaction. The removal of the residual enzyme or inactivation of the enzyme can be performed by the usual methods, but such processes are not necessarily required.

The decomposed materials after proteolysis contains undecomposed residues and coloured materials and other substances. These are removed by filtering, absorption or other suitable methods, and if necessary the concentration is adjusted. In the extracted material, water-soluble high polymers are contained and must be removed for the preservation and clinical safety of the product. There are many methods of deproteinization such as precipitation, absorption and salting out. One preferable method, using iso-electronic precipitation, is to adjust the pH of the solution to 4—5 and then to remove the precipitated proteins. The extract solution (i.e. the agent of the invention) obtained by such procedure is a colourless to yellowish transparent liquid and has properties which are described below.

When this extract solution is used for medical purposes for instance, as injection, analgesic agents and ingredients to make it isotonic may be added and bacterial filtration is performed and the material is filled into ampoules and then sterilized. The inventors clinically tested the said injections prepared as above with patients, who desired to be treated, and observed remarkable effects.

The agent (extract) according to the invention is further described below.

(a) Clinical dosage and method of administration

Dosage of the agent prepared as described in Example 1 below for administration by injection varies with the age of patients, but effects can be expected normally by the administration of a single dose of 2—5 ml of the agent, 1—4 times a week, and especially a good result can be obtained by administering a single dose of 2—5 ml of the agent 2—3 times/week. Satisfactory results can be expected by both subcutaneous and intramuscular injection.

(b) Acute Toxicity

DD strain male mice (17 0 22 g of weight, of 5 weeks) were housed beforehand in constant conditions at $24 \pm 1°C$ with a relative humidity of $55 \pm 5\%$. Injections consisting of the agent of this invention, were administered to groups consisting of 10 animals, subcutaneously and intraperitoneally and the animals were examined 48 hours later and one week later for clinical symptoms and mortality. The dose of injection was increased up to 150 ml/kg on both methods of administration, but no animal died and no unusual clinical symptoms were observed. Therefore the toxicity of this substance is thought to be very low.

(c) The human umbilical cord extract as prepared below showed the following physical characteristics.

(i) The pH is $6.5 \pm 0.22$.

(ii) Weight of dried sample.

When 1.0 ml of the extract of this extract, adjusted to a specific gravity of $1.016 \pm 0.004$, is dried at 100°C for 48 hours and weighed, the weight of the dried sample is 22.3—40.1 mg.

(iii) Ultraviolet absorption spectrum

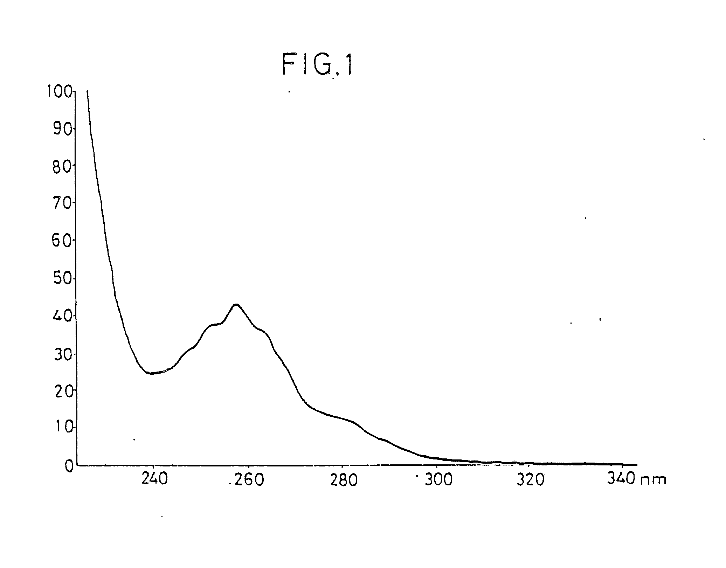

The extract has a maximum absorption in the ultraviolet spectrum at 257 nm (refer to Figure 1).

(iv) $\alpha$-amino nitrogen content

The $\alpha$-amino nitrogen content of 100 ml (1.016 sp.g) of the extract titrated by formol titration is $53.80 \pm 15.2$ mg.

(v) Total nitrogen content

Applying the method of nitrogen determination of the Japanese Pharmacopeia, the total nitrogen content of the extract is $185 \pm 30.6$ mg/dl (sp.gr. 1.016).

(vi) Amino acids

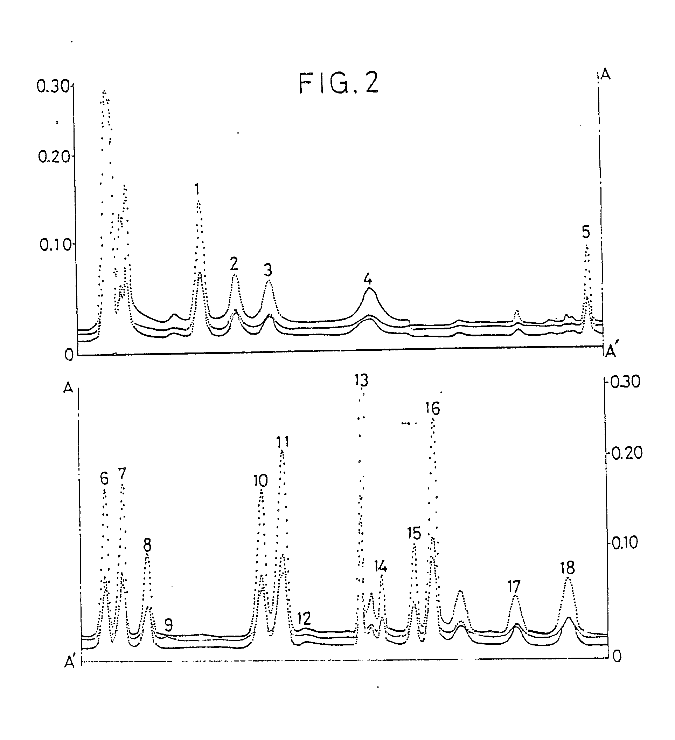

1 ml of 1% picric acid was added to 1 ml of the extracted (sp.gr. 1.016), shaken and mixed, and after a whole day and night, was centrifuged, and the supernatant was poured through a column of ion exchange resin (Dowex 2—8 Cl — the word "Dowex" is a Trade Mark) to remove picric acid and used as a sample. Refer to Figure 2 (Hitachi KLA-5 Amino acid Analtzer, resin in column; Hitachi custom resin No. 2618, Solution; 0.2 N sodium citrate buffer).

(vii) Hexosamine Content

1 ml of 4 N HCl was added to the extract and the extract was enclosed in a glass vial and heated for 15 hours and then neutralized with 10 N NaOH using phenolphthalein as indicator, and adjusted to 5 ml. Then 1 ml of this solution was taken and was examined for hexosamine by Elson and Morgan's method. The hexosamine content was $340.0 \pm 15.2$ $\mu$g glucosamine HCl/ml.

The autonomic imbalance treating agent of this invention is administered most effectively by injection and using this method of administration, and clinically tested with autonomic imbalance patients. There were about 500 autonomic imbalance patients who desired to have therapy with this agent. Of these patients 320 were studied over the last three year period. Most of the outpatients were over the age of 20 and female, 200 out of the 320 patients being women. The largest proportion of the patients were in their forties (age in years) and the next largest proportion of the patients were in their twenties and thirties. (The number of people in their twenties suffering from autonomic imbalance is increasing). The occupation of the patients were various: company employees were largest in number and the next largest in number were housewives.

In the past 5 years, about 85% of the patients were effectively treated by means of the agent of the invention, of the 320 patients treated in the last 3 year period, the agent was effective for 90% of the patients. The agent was about 80% effective in females and about 93% effective in males.

The effectiveness of the agent with regard to various complaints were as follows:

For the symptoms of autonomic imbalance (headache, dullhead, ease of being tired, stiff shoulders, insomnia, gastro-intestinal disturbance, chilly extremities, dizziness and asthenopia) more than 90% for gastrointestinal neurosis and pharyngolaryngeal neurosis also more than 90%, and for climacteric disturbance and orthostatic disturbance more than 85% effectiveness was observed. For cyclic vomiting, nocturnal enuresis and neurotonia more than 80%, for other various autonomic imbalance more than 80%, for autonomic imbalance caused by conversion or somatization of e.g.

4

anxiety and strain more than 82%, and for other autonomic imbalances and illness combined with autonomic imbalance more than 75—90% effectiveness was indicated.

A dose of 2—5 ml administered once to three times a week, alleviated the symptoms markedly after an average of 10—15 administrations. Some patients recovered after 6 or less administrations. (One patient recovered after only one administration). Symptoms originating in autonomic imbalance, such as ease of being tired, feverish feeling, sensitiveness to cold, sweating, emaciation, insomnia, dizziness, headache, dullhead, stiff shoulders, numbness of extremities, palpitation, breathlessness, oppression in chest, loss of appetite, thirst, nausea, vomiting, asthenopia, abdominal pain, diarrhea and constipation were improved markedly and other symptoms also were alleviated. Thus the clinical usefulness of the agent according to the invention was demonstrated.

Further no adverse reaction seemingly related to the agent was observed. As we have seen, human umbilical cord extract has a marked effect in a broad range of patients from infants to adults. In view of these facts, the agent of the invention, which is a clinically useful one with high effectiveness and extremely high safety, can be said to be a boon to patients who are suffering from autonomic imbalance.

The following examples of 21 clinical cases illustrate the pharmaceutical effects of the agent of the invention. By "PC-007" is understood injections (i.e. injectable compositions) consisting of the autonomic imbalance treating agent of the invention. 30 other cases of clinical administration are shown later as TABLE OF CASE REPORTS, to illustrate the clinical effects of the agent.

Case 1

A.H. female, autonomic imbalance with fits of migraine.

(1) Age of first medical examination: 20 years old

(2) Anamnesis: none

(3) Family history: The patient's sister had the same symptoms and was treated and her symptoms were alleviated by injection PC-007 in our hospital.

(4) Case history: Since the age of 18 she had a defective visual field following a fit of headache with nausea. The headache was usually unilateral but sometimes bilateral. She was examined at a ophthalmologist and an ophthalmologic section in a general hospital but was told to have nothing ophthalmologically abnormal. July 30, 1975, while looking at the television she lost partially sight of visual field for about 30 minutes (defect of visual field). Following that, she had a severe dextrous migraine and nausea and took analgesics. First medical examination in this hospital was August 5, 1975.

(5) Condition at first examination: Constitution; middle-sized, Nutrition; good, Blood pressure; (100/60), Urinary protein; ( $\pm$ ), Urinary glucose; (—), Urobilinogen (N+), Blood sedimentation rate; (7 mm/hr), CRP; (—), GOT, GPT, ALP, ZTT, Cholesterol, TP, A/G rate, Electrolyte and so on; nothing abnormal. Chest X-ray and ECG nothing unusual. In response to detailed inquiry, she complained of difficulty in getting up in the morning, stiff shoulders, fatigue of eyes, impatience, irritability and chilled extremities. The CMI test (Cornell Medical Index; Kanehisa Takuya et al: Cornell Medical Index, illustration and data, Sankyobo, 1972) showed Group IV.

From the aforesaid symptoms the case was diagnosed as autonomic imbalance with fits of migraine.

(6) Course of treatment (since treated in our hospital): Analgesics were administered when fits of headache occurred and PC-007 was injected from August 8 to the last day of November, with a dose of 3 ml twice a week, total 30 times. During this period she had only one fit, and since the end of the treatment all of the equivocal complaints disappeared. This is a case where the agent of the invention showed an outstanding effect.

Case 2

S.K. female, autonomic imbalance with abdominal symptoms.

(1) Age of first medical examination: 30 years old

(2) Anamnesis: none

(3) Family history: Mother has hypertension and diabetes.

(4) Case history: Had headache, nausea and dizziness for one month before coming to out hospital and had been examined at a clinic and general hospital, but her symptoms could not be eased and she arrived at our hospital on April 16, 1977.

(5) Condition at first examination: Constitution; middle-sized, Nutrition; good, Anaemia not observed, Physical condition; has light tenderness in the left epigastrium but nothing besides unusual. Neuropathic, nothing unusual such as head retraction, morbid reflects and abnormal ocular movement. Clinical test; Blood pressure (107/70); Urine (normal); Blood sedimentation rate (5 mm/hr); Number of leucocytes, 5600; Number of erythrocytes, 430 x $10^4$; Hemoglobin, 85%; and nothing unusual. Also nothing abnormal was observed in GOT, GPT, ALP, ZTT, Cholesterol, TP, A/G rate, Chest X-ray, Simple abdominal X-ray and Gastro radioscopy. In response to detailed inquiry, she complained that she had stiff neck and shoulder and felt heavy in her legs and arms, that she usually had a heavy head, was easily tired in work, and found it hard to get up, CMI test indicated group IV.

5

From aforesaid symptoms was diagnosed autonomic imbalance with abdominal symptoms.

(6) Course of treatment (since arrival in our hospital): The therapy with PC-007 injections beginning from April 22, 1977 continued until the end of the following June, with a dose of 2 ml, twice weekly, 18 times in all.

The results: at the beginning of the therapy analgesics were administered for headache, but after a week of administration they became needless and nausea lessened and equivocal complaints such as exhaustion of whole body gradually improved. Two months later at the end of June, the symptoms had almost disappeared. This case shows the outstanding effect of the agent of the invention.

Case 3

K.K. male, autonomic imbalance with complaint of palpitation.

(1) Age of first medical examination: 46 years old

(2) Anamnesis: gastric ulcer at the age of 23.

(3) Family history: Father, hypertension and brother, tuberculosis.

(4) Case history: Since he had bad times in trade after the "Oil shock", began to have ear noise, and 6 months before arrival at our hospital had palpitation, loss of appetite and insomnia, and was treated in several clinics and hospitals, but with no distinct recovery. On July 13, 1977 he arrived at our hospital.

(5) Condition at first examination: Constitution, slender; Nutrition, well fed; Physical conditions, nothing unusual, Clinical tests: Blood pressure (107/75), Urine (normal); Blood sedimentation rate (4 mm/hr); GOT, GPT, ALP, ZTT, Cholesterol, TP, A/G rate, Electrolyte, and Chest X ray indicated nothing unusual. ECG step test was also in normal range. To the detailed inquiry, there were complaints such as dullhead as if staying up all night, unsteadiness of body, pain in back and abnormal sweating for the previous one and a half years and cool extremities. CMI test indicated group III.

From the aforesaid symptoms was diagnosed autonomic imbalance with complaints of palpitation.

(6) Course of treatment (since arrival at our hospital): Since July 13, 1977 Nervenruh-forte (inhibitor of central nervous system, with antispasmodic and antalgic effects) was administered 6 Tab/day for 6 weeks, but with no distinct recovery, so Bellergal (autonomic imbalance agent) 3 Tab/day was administered for one week but there was no recovery. From August 13, 1977 to September 15, PC-007 5 ml of dose, twice a week, 8 times in all was administered.

The results: Palpitation disappeared around September 1, and the pain in back also disappeared and colourful equivocal complaints had almost disappeared after one month of treatment, and proved the outstanding effect of the agent of the invention.

Case 4

Y.O. female, autonomic imbalance caused by stress.

(1) Age of first examination: 41 years old

(2) Anamnesis: none

(3) Family history: none

(4) Case history: She lost her eldest son in a car accident in May 1976 and experienced dizziness since about July and when tired, had headache and nausea. Arrived at our hospital on September 9, 1976.

(5) Condition at first examination: Constitution, middle-sized; Nutrition, well fed; anaemia, not observed; Physical condition, nothing unusual. Clinical tests: Blood pressure (115/67), Urine (normal) Blood sedimentation rate (3 mm/hr); Peripheral hemogram, Biochemical blood tests, Chest X ray, EKG and Funduscopy indicated nothing unusual. Neuropathic symptoms were also not observed. In response to detailed inquiry, she complained of severe dizziness and fatigue of the whole body especially severe in the morning and also thirst and constipation. CMI test indicated group IV. She was diagnosed as a case of autonomic imbalance caused by the stress of her son's death.

(6) Course of treatment (since arrival at our hospital): Betahistine mesylate 6 Tab/day was administered and intramuscular injection of 5 ml PC-007 twice a week, 8 times in all. During the treatment dizziness considerably decreased and almost all equivocal symptoms such as feeling of exhaustion, dullhead, stiff shoulders and others disappeared.

Case 5

I.T. male, Gallbladder dyskinesia caused by autonomic imbalance

(1) Age of first examination: 45 years old

(2) Anamnesis: none

(3) Family history: none

(4) Case history: Since the middle of August, 1977 had feeling of epigastric dilatation, nausea, fatigue of the whole body, dizziness and heavy feeding from the epigastrium through the chest, and arrived at our hospital on September 17.

(5) Condition at first examination: Tenderness at the right upper abdomen, anaemia or jaundice not observed, liver and spleen not palpable. Clinical tests: Blood pressure (138/90); Urine (normal);

Blood sedimentation rate (16 mm/hr); GOT, GPT, LAP, ZTT, TP, A/G rate and Cholesterol indicated nothing unusual, also Chest X ray, simple abdominal X ray, Gastro-radioscopy and EKG indicated nothing unusual. By cholecystograph the picture was clear and no gall stone or dilatation of bile duct was observed. CMI test indicated group IV. In response to detailed inquiry, he complained of stiff shoulders, asthenopia, dizziness and feeling of easily being tired.

From aforesaid symptoms, the case was diagnosed as dyskinesia caused by autonomic imbalance.

(6) Course of treatment (since arrival at our hospital): 5 ml of PC-007 injection was administered three times a week, for three weeks, 9 times in all. As a result, equivocal complaints decreased and the chief complaint of epigastric dilatation and nausea disappeared and, as the symptoms by palpation became normal, the treatment ended on October 15. Improvement of equivocal complaints is thought to be the effect of the agent of the invention.

## Case 6

Y.D. male, nocturnal unuresis

(1) Age of first examination: 11 years old

(2) Case history: Boy of 5th year class of primary school still wetting the bed every night and cannot go with schoolmates to camp or seaside school. Since in the third year class had consulted various medical doctors, pediatrists, pediatrics of general hospitals and neurologists. The parents wishing to let him go on the school excursion, brought him to our hospital.

(3) Course of treatment: Intramuscular injection of PC-007 5 ml was administered twice a week (5 times of administration, after two weeks) the nightly bed wetting was reduced to about twice a week and after 14 injections it was reduced to once in ten days, after 5 weeks of administration, injection was reduced to once a week, and bedwetting almost disappeared. He could go to the 6th year class spring excursion.

## Case 7

T.F. female, autonomic imbalance

(1) Age of first examination: 47 years old

(2) Anamnesis: none

(3) Case history: About 3 months before her arrival at our hospital, she had colourful equivocal complaints of headache, dizziness, sensitivity to cold, weariness and had consulted clinics and hospitals and was treated, but with no distinct discovery, and arrived at our hospital on October 21, 1976.

(4) Condition at first examination: Constitution, middle-sized; Nutrition, well fed; Nothing unusual in physical condition. Clinical tests: Blood pressure (108/72), Urine (normal) Blood sedimentation rate (5 mm/hr), peripheral hemogram, GOT, GPT, ALP, ZTT, Cholesterol, TP, A/Grate, Chest X ray, Simple abdominal X ray, Gastro-radioscopy and Electrolyte indicated nothing unusual. In response to detailed inquiry, other than the aforesaid complaints there were lumbago, palpitation, breathlessness, and dullhead. CMI test indicated group II.

From aforesaid symptoms the case was diagnosed as autonomic imbalance.

(5) Course of treatment (since arrival at our hospital): Treatment with PC-007 injection started from October 27, 1976, till December 10, dose of 2 ml, twice a week 12 times in all was administered. As a result, the strong complaints at the beginning of the therapy, headache, stiff shoulders, and sensitivity to cold had decreased and after one month of the therapy, almost all colourful complaints disappeared, so that treatment was ended after 12 administrations. Thereafter no complaints were observed. This is an outstanding case of effectiveness of the agent of the invention.

## Case 8

H.K. female, abdominal symptoms by autonomic imbalance

(1) Age of first examination: 53 years old

(2) Anamnesis: none

(3) Case history: She was a nurse in a surgical clinic for a long time and 5 years ago she began to feel languid and especially had headache, nausea, vomiting, stiff shoulders, and easily got tired during work, and was often absent from her workplace. During this period she received various treatments but did not recover. She arrived at our hospital on November 21, 1975.

(4) Condition at first examination: Constitution, slender; Nutrition, well fed; Blood pressure (120/78); Urine (normal); Blood sedimentation rate, (4 mm/hr); Peripheral hemogram, ALP, GOT, GPT, TP A/G rate, Cholestrol, ZTT, Electrolytes, Chest X ray and Gastro-radioscopy indicated nothing unusual. In response to detailed inquiry there were complaints other than the aforesaid symptoms such as dizziness, lumbago, palpitation, breathlessness and thirst. CMI test indicated group IV.

From the aforesaid results the case was diagnosed as abdominal symptoms due to autonomic imbalance.

(5) Course of treatment (since arrival at our hospital): The therapy with the agent began on September 27, 1975 and continued until the middle of December, 2 ml of injection was administered once or twice a week, 13 times in all. The main complaint markedly decreased after 2 weeks of administration. After that, the agent was administered once a week until in the middle of December the symptoms disappeared and the therapy was stopped. This showed an outstanding effect of the agent of the invention.

## Case 9

Y.T. male, periodic vomiting

(1) Age of first examination: 5 years old

(2) Case history: The mother of the patient is nervous and he occasionally developed fever and was also nervous in character. She said that 8 days before arrival at our hospital he vomited without any reason and 3 days later had vomiting and abdominal pain, after that, he had repeated vomiting, looked limp and was depressed, ill-humoured and had a tendency to constipation.

(3) Condition at first examination: Constitution, middle-sized; Malnutrition; Somewhat dehydrated; Complexion pale; Lip dried; Palpitation; Tenderness in the region of navel and abdomen. Also had foul breath with smell of acetone. Similar symptoms had occurred 6 times in 2 years, and 4 times he had entered hospital and had been treated. Clinical tests: Urine, strong acetonuria, Urobilinogen ($\pm$), Blood sugar, low; Blood sedimentation rate, (3 mm/hr); Stool (normal).

From the aforesaid symptoms the case was diagnosed as periodical vomiting and he was taken into our hospital.

(4) Course of treatment (since arrival at our hospital): Vomiting was treated symptomatically and PC-007 injection 2 ml was administered 3 times in a week and consequently the occasional abdominal pain ceased, appetite improved, colour was regained and he recovered his strength. After that 2 injections were administered weekly, 9 injections in all, and after that no sympton was observed for one year. This is an effective case of the agent of the invention.

## Case 10

O.T. female, psychogenic autonomic imbalance

(1) Age of first examination: 14 years old

(2) Anamnesis: none

(3) Family history: Since the 5th year class of primary school had orthostatic disturbance and received various therapies but did not improve and was frequently absent from school, and since in the second year of middle school had complaints such as fatigue of whole body, dullhead, nausea, dizziness, insomnia, abdominal pain and others, and scarcely went to school. She received treatment at several hospitals but without good results and arrived at our hospital in May, 1976.

(5) Condition at first examination: Constitution, slender; Nutrition, well developed; Anaemia not observed. Clinical test: Liver function tests such as GOT, GPT, and others, Gastro and Intestinal radioscopy, EEG and Chest X-Ray, every test showed nothing unusual. The mecholyl test as a test for autonomic function indicated sympathetic hypotonia. In response to detailed inquiry, she complained of other symptoms other than the aforesaid such as thirst, breathlessness and headache, and CMI test indicated group IV. Y—G test indicated social maladjustment, emotional instability and introvertness. Her family's circumstances were that she was blindly loved by her family when a child but since she had been examined in a university hospital and was diagnosed as psychogenic, her family became severe and since then she became to show colourful complaints of headache, abdominal pain and others and frequently stayed away from school.

From the aforesaid symptoms the case was diagnosed as psychogenic autonomic imbalance.

(6) Course of treatment (since arrival at our hospital): Since the middle of May 1976, autogenic training was given and the therapy with the agent of the invention was begun. Two ml of injection twice a week, 16 times in all was given. As a result 3 weeks after the beginning of administration the complaints since the opening of the therapy markedly decreased and in the middle of July, the symptoms almost disappeared and the treatment was stopped, and she was going to school cheerfully.

## Case 11

M.S. female, psychogenic autonomic imbalance.

(1) Age of first examination: 13 years old

(2) Anamnesis: none

(3) Family history: Mother, autonomic imbalance

(4) Case history: Since she was in the 6th year class of primary school she had complained of chilling of extremities, dizziness, dullhead and headache, and received various therapies, but did not improve and frequently was absent from school. She arrived to our hospital to be examined in June, 1976.

(5) Condition at first examination: Constitution, middle-sized; Nutrition, well developed. Clinical tests: Blood pressure (108/65), Urine (normal), Blood sedimentation rate and peripheral hemogram were both normal. Liver function, Chest X ray, EKG and EEG, were all normal. The mecholyl test for autonomic function indicated sympatheticotonia. In response to detailed inquiry, there were complaints other than the aforesaid such as numbness of extremities, insomnia and CMI test indicated group IV. The patient was examined with her mother who had a strong tendency to hypochondria and had been diagnosed to have autonomic imbalance and had been treated for several years.

After all, the patient was thought to be influenced by the forceful suggestion of her hypochondric mother and was diagnosed as having psychogenic autonomic imbalance.

(6) The course of treatment (since arrival at our hospital): Since the middle of 1976, the treatment with PC-007 was started, and till the middle of July, 2 ml of injection, twice a week 10 times in all, was administered. As a result, the main complaints from the beginning of the therapy, decreased markedly after 2 weeks of therapy, 4 weeks later the symptoms disappeared and the therapy was stopped. The patient's mother was also treated with PC-007, and she also showed outstanding improvement. Thus both the mother and daughter were cured by the agent of the invention.

### Case 12

M.T. male, essential autonomic imbalance (hypersensitive intestinal syndrome)

(1) Age of first examination: 16 years old

(2) Anamnesis: none

(3) Family history: none

(4) Case history: Since in the second year class of the middle school, complained of fatigue and occasionally indicated abdominal pain and constipation. After entering high school these symptoms continued, and complexion was bad, he lacked constitutional power and could not attend physical training, also could not follow school work. During the period received various treatments, but did not show improvement, and arrived at our hospital in October 1974.

(5) Condition at first examination: Constitution, somewhat slender, Anaemia, not observed. Clinical examination: Blood pressure (110/70), Urine, Intestinal radioscopy, both natural; Liver tests, peripheral hemogram, Chest X ray and others showed nothing unusual. In response to detailed inquiry, he complained of abdominal discomfort, dilatation, thirst, palpitation and feverish feeling. CMI test indicated group II with various physical complaints.

From the aforesaid, the student was thought to be extraordinary sensitive to abdominal pain and constipation, and was diagnosed as a case of essential autonomic imbalance (hypertensive intestinal syndrome).

(6) Course of treatment (since arrival at our hospital): Therapy with PC-007 injection was started from the end of October and till the beginning of December, 2 ml of injection, twice a week, 12 injections in all were administered. As a result, 2 weeks later complaints began to decrease and abdominal pain and constipation markedly improved and in the end of November abdominal pain ceased to occur and the therapy was stopped in the beginning of December. This case also showed the outstanding effect of the agent of the invention.

### Case 13

D.S. female, psychogenic autonomic imbalance with vomiting as chief complaint.

(1) Age of first examination: 20 years old

(2) Anamnesis: none

(3) Family history: none

(4) Case history: Since February 1975 had nausea with vomiting and in addition to that, complaints of asthenopia, abdominal disorder, easily being tired, and sweating, and the day after drinking had severe nausea and vomiting, and the other symptoms also appeared strongly. She was treated in various hospitals and clinics but did not improve. She arrived at our hospital in November, 1975.

(5) Condition at first examination: Constitution, middle-sized; Nutrition, well developed. Clinical tests: Blood pressure (118/73), Gastric X ray examination, Gallbladder X ray, nothing unusual; Urine, Liver function, Pancreas function, Peripheral hemogram, Chest X ray, nothing unusual. In response to detailed inquiry, she complained of headache, dullhead, sensitive to cold, dizziness and insomnia. The mecholyl test indicated a tendency of parasympatheticotonia CMI test indicated group IV.

Complicated family circumstances and disappointed love should have made her distrust the male sex, and the conversion of this may have caused vomiting and other symptoms. This case was diagnosed as psychogenic autonomic imbalance with vomiting as chief complaint.

(6) Course of treatment (since arrival at our hospital): The treatment with PC-007 was started from November 19, 1975. 5 ml of injection, twice a week, 10 injections in all was administered. During the second week the symptoms markedly improved, at the end of 4th week most of the complaints disappeared and the chief complaints such as nausea and vomiting ceased. The therapy was stopped in the 5th week. This is a case that shows the efficacy of the agent of the invention.

### Case 14

T.Y. female, psychogenic autonomic imbalance with tension and anxiety.

(1) Age of first examination: 20 years old

(2) Anamnesis: none

(3) Family history: Mother died 3 months before of uterine cancer.

(4) Case history: About 3 months before her arrival at our hospital, complaints of stiff shoulders, numbness of extremities, insomnia, and loss of appetite appeared. Complexion was pale, was easily tired, and she was frequently in bed.

She was examined on June 10, 1975 in our hospital.

(5) Condition at first examination: Constitution, middle-sized, Nutrition, somewhat poor; Colour, pale; Clinical tests: Chest X ray, EKG, Gastric X ray, were both normal, GOT, GPT, and other liver tests, Urine, Serum, Electrolytes, peripheral hemogram and other tests showed nothing unusual. In response to inquiry, there were more complaints. The CMI test indicated a tendency to neurosis and she was classified in group IV. As mentioned above, her mother had entered hospital 10 months before for uterine cancer and died 3 months ago, during the period she had been nursing and the various symptoms began to appear after that.

From aforesaid results, it was thought that the strain and anxiety caused by her mother's death, and anxiety of the future caused by losing her mother had become the cause of the complaints and the case was diagnosed as psychogenetic autonomic imbalance with tension and anxiety.

(6) Course of treatment (since arrival at our hospital): The treatment with PC-007 injection began from June 18, till the middle of August, twice a week, 2 ml, 16 injections, in all. As a result, from the third week she markedly improved and in August almost every symptom disappeared and her colour became good.

Case 15

K.T. female, psychogenic autonomic imbalance

(1) Age of first examination: 45 years old

(2) Anamnesis: none

(3) Family history: none

(4) Case history: At the age of 40, there were complaints of dizziness, dullhead, stiff shoulders, lumbago and ease of being tired and she was examined at several clinics and hospitals, but did not improve, and arrived at our hospital on February 25, 1975.

(5) Condition at first examination: Constitution, middle-sized, Nutrition well developed; Para III; Menstruation regular. Clinical tests: Blood pressure (128/80); Urine (normal); Liver function tests, Serum electrolytes, Chest X ray, EEG and other tests showed nothing unusual. In response to detailed inquiry, there were more complaints of constipation, abdominal disorder and dilatation, asthenopia, thirst and numbness of extremities. CMI test showed physical sign and group IV.

On inquiry into her family circumstances she revealed that at the age of 40, her husband had a secret love affair with a woman and she met the woman and had a heavy shock, since then her distrust of her husband had increased, and the aforesaid symptoms strongly appeared. From the aforesaid results the case was diagnosed as the so-called climacteric psychogenic autonomic imbalance.

(6) Course of treatment (since arrival at our hospital): Since March 5 the treatment with PC-007 was started and till the beginning of June, twice a week, an injection of 5 ml, 22 injections in all, was administered. As a result, after 5 weeks of treatment there was a marked improvement, the husband was called and told that the cause of the sickness of his wife was his love affair and was persuaded to separate from the woman. Afterwards in the end of May the symptoms almost disappeared and the administration was stopped. This case shows the effectiveness of the agent, of the invention.

Case 16

U.H. female, Climacteric disturbance

(1) Age of first examination: 50 years old

(2) Anamnesis: none

(3) Family history: none

(4) Case history: Since about the age of 47 she had complained of stiff shoulders and headache and others and was diagnosed at a clinic as having climatic disturbance and was treated but did not: get well, and arrived at our hospital on January 21, 1976, to be examined.

(5) Condition at first examination: Constitution, middle-sized, Nutrition well developed, Anaemia not observed and nothing unusual in physical symptoms. Clinical tests: Blood pressure (132/81), Blood sedimentation rate (6 mm/hr), Urine (normal), Peripheral hemogram, Liver function test, Serum electrolyte, EEG and Chest X-ray showed nothing unusual. In response to detailed inquiry, she complained of more symptoms other than the aforesaid such as sensitiveness to cold, palpitation, breathlessness and numbness of extremities. CMI test showed physical signs and indicated group II.

From the aforesaid, the case was diagnosed as climacteric disturbance.

(6) Course of treatment (since arrival at our hospital): Since January 28, 1976, the treatment with the agent of the invention was started and till the beginning of March, twice a week, 5 ml of injection, 10 injections in all were administered. As a result, the complaints at the beginning of the treatment markedly decreased, and at the end of February they had almost disappeared so that the administration was stopped.

Case 17

S.Y. female, autonomic imbalance caused by hysterectomy.

(1) Age of first examination: 42 years old

(2) Anamnesis: At 41 years of age received hysterectomy for myoma of uterus.

(3) Family history: none

(4) Case history: A short time after the operation there were equivocal complaints of stiff shoulders, ease of being tired, a hot fit, sensitiveness to cold, lumbago, irritancy and she had a first examination on February 10, 1976.

(5) Condition at first examination: Constitution, somewhat slender, nutrition somewhat poor, Anaemia not observed, Clinical tests: Blood pressure (125/78), Urine (normal), Blood sedimentation rate, Liver function tests, Chest X ray, EEG, Serum electrolytes, and Peripheral hemogram showed nothing unusual. In response to detailed inquiry, she complained of hyposexualitas, sweating, ear noises and dizziness and CMI test indicated group IV.

From the aforesaid, this case was diagnosed as autonomic imbalance caused by hysterectomy.

(6) Course of treatment (since arrival at our hospital): The treatment with PC-007 started from February 18 and till the beginning of April twice a week, 2 ml, 14 injections in all were administered. As a result from the third week a marked improvement was shown and at the beginning of April the symptoms disappeared and the treatment was ended.

Case 18

I.H. female, post capital trauma autonomic imbalance.

(1) Age of first examination: 29 years old

(2) Anamnesis: none

(3) Family history: none

(4) Case history: She received capital trauma by traffic accident and entered hospital, and the trauma healed, but after that, occasionally equivocal complaints of headache, dullhead, pain in the neck and stiff shoulders appeared and she was examined in a general hospital and treated but did not recover and arrived at our hospital on April 6, 1976.

(5) Condition at first examination: Constitution, Large; Nutrition well developed. Clinical examination: Blood pressure (131/76), Blood sedimentation rate (5 mm/hr); Urine (normal); EEG, Liver function tests, Chest X ray, Serum electrolytes and other general tests showed nothing unusual. In response to detailed inquiry, every complaint was recognized as a symptom of autonomic imbalance.

From the aforesaid the case was diagnosed as post capital traumatic autonomic imbalance caused by traffic accident.

(6) Course of treatment (since arrival at our hospital): Treatment with PC-007 was started since April 14, and twice a week, 5 ml of injection, 26 injections in all were administered. As a result, the strong equivocal complaints markedly decreased in the fifth week and in the middle of June almost all of them disappeared.

Case 19

S.A. male, essential autonomic imbalance by abnormal constitution.

(1) Age of first examination: 24 years old

(2) Anamnesis: From his childhood he has an orthostatic syncope that suggests autonomic imbalance which grew worse seasonally.

(3) Family history: mother, hypotension

(4) Case history: The patient was working in a trading company, and about 5 months previously he became busy in foreign trade, and meal times became irregular and he could not have enough sleep. From about that time equivocal complaints such as dullhead, dullness of whole body, stiff shoulders, and loss of appetite appeared. He arrived and was examined in our hospital on November 24, 1976.

(5) Condition at first examination: Constitution, slender; Nutrition well developed; Anaemia not observed. Clinical tests: Blood pressure (119/75), Blood sedimentation rate (5 mm/hr), Liver function tests, Urine, Chest X ray, EEG and Peripheral hemogram, showed nothing unusual. In response to detailed inquiry, equivocal complaints which seemed to be symptoms of autonomic imbalance other than the aforesaid were revealed. Since before, he had a complex of his slender constitution which resembles his mother, and this is thought to have sometimes caused the equivocal complaints, and CMI test indicated group II.

From the aforesaid the case was diagnosed as essential autonomic imbalance caused by abnormal constitution.

(6) Course of treatment (since arrival at our hospital): PC-007 treatment began from November 30, twice a week, 5 ml, 8 injections in all, until the end of December. As a result, a marked improvement was observed in the second week and the administration was stopped. This is an outstanding case of the effectiveness of the agent of the invention.

## Case 20

S.K. male, discomfort in throat and general equivocal symptoms.

(1) Age of first examination: 25 years old

(2) Anamnesis: none

(3) Family history: none

(4) Case history: For 5 months before arrival at our hospital he strongly complained of discomfort in the throat and also had general equivocal symptoms of stiff shoulders, chilly extremities, asthenopia and ease of being tired, and was treated in general hospital but was not improved, and was examined in our hospital on April 2, 1974.

(5) Condition at first examination: Constitution, middle-sized; Nutrition, somewhat poor; Anaemia not observed. Otolaryngoscopy nothing unusual, Cervical X ray nothing unusual; the results of other clinical tests such as blood pressure (121/750), Urine (normal), Liver function tests, peripheral hemogram, serum electrolytes, Pancreas function tests, Chest X-ray and EEG were all normal. In response to detailed inquiry, he complained that the discomfort in the throat was in the form of foreign body sensation, and he complained of dryness, strictured feeling and pain in the throat, and other various general equivocal symptoms of headache and insomnia, CMI test indicated Group II.

From the aforesaid results the case was diagnosed as general equivocal symptoms with discomfort in throat.

(6) Course of treatment (since arrival at our hospital): PC-007 therapy was started from April 9, and twice a week 5 ml, 8 injections in all, till the beginning of May, were administered. As a result, in the last half of the second week the discomfort in the throat markedly decreased and at the end of April almost all the symptoms disappeared, so that administration was stopped.

## Case 21

K.M. female, equivocal syndrome with gastroptosis.

(1) Age of first examination: 42 years old

(2) Anamnesis: none

(3) Family history: none

(4) Case history: About 5 months before her arrival at our hospital, she had colourful complaints of nausea, vomiting, heartburn, loss of appetite, eructation, constipation and stiff shoulders and she was examined in a general hospital and diagnosed as having gastroptosis and received various treatments, but did not get well, and was examined in our hospital on July 2, 1974.

(5) Condition at first examination: Constitution, slender; Nutrition somewhat poor, Anaemia not observed. Clinical tests: Gastro X ray (gastroptosis was observed), Blood pressure (118/75); Urine (normal). Other general tests showed nothing unusual. In response to detailed inquiry, she had complained of, in addition to the aforesaid symptoms, dizziness, irritability and anxiety, insomnia and chilly extremities CMI test indicated group IV.

From the aforesaid results, the case was diagnosed as equivocal syndromes with gastroptosis.

(6) Course of treatment (since arrival at our hospital): PC-007 treatment was started from July 10, and twice a week, 5 ml, 12 injections in all were administered. As a result, in the fifth week, the symptoms markedly improved and the colour became good.

## TABLE OF CASE REPORTS

| Case Report No. | Name | Age and Sex | Diagnosis | Complaints | CMI | Complication | Period of treatment |
|---|---|---|---|---|---|---|---|
| 1 | K.K. | 49 F | Climacteric disturbance | loss of appetite, fatigue | IV | none | 3 M |
| 2 | M.S. | 26 F | Autonomic imbalance | stiff shoulder, ease of being tired | III | " | 2 M |
| 3 | A.H. | 42 F | " | " | IV | " | 7 M |
| 4 | S.M. | 42 F | " | stiff shoulder, dizziness | II | " | 2 M |
| 5 | S.M. | 36 F | " | dizziness | IV | " | 10 D |
| 6 | S.M. | 45 F | " | ear noise, dizziness | IV | " | 1 W |
| 7 | T.Y. | 49 F | Climacteric disturbance | stiff shoulder | III | " | 12 M |
| 8 | K.K. | 47 F | Autonomic imbalance | stiff shoulder, ease of being tired | II | " | 1 M |
| 9 | S.N. | 37 F | " | headache, dull head | II | hypertension | 12 M |
| 10 | K.A. | 53 F | " | nausea, vomiting | IV | none | 5 Y |
| 11 | T.I. | 45 M | Autonomic imbalance (Biliary dyskinesia) | upper abdominal dilatation, nausea | IV | Biliary dyskinesia | 1 M |
| 12 | H.O. | 43 F | Autonomic imbalance | headache | IV | none | 1 Y |
| 13 | M.K. | 35 F | " | dizziness | III | " | 1 W |
| 14 | M.S. | 49 F | Neurocirculatory asthenia | palpitation | IV | " | 1 M |
| 15 | Y.S. | 52 F | Climacteric disturbance | sensitiveness to cold | IV | " | — |

0 012 774

| Case Report No. | Name | Age and sex | Diagnosis | Complaints | CMI | Complication | Period of treatment |
|---|---|---|---|---|---|---|---|
| 16 | Y.O. | 29 F | Autonomic imbalance | abdominal disorder and dilatation | III | none | 1 Y |
| 17 | T.Y. | 41 F | " | dizziness, headache | IV | " | 2 M |
| 18 | N.M. | 34 F | " | dizziness | II | " | — |
| 19 | K.K. | 49 M | " | stiff shoulder | IV | " | 1 M |
| 20 | K.K. | 46 M | " | palpitation | III | " | 1 Y 6 M |
| 21 | I.O. | 45 F | " | headache | II | " | 2 Y |
| 22 | K.O. | 44 F | " | constipation | IV | " | 1 W |
| 23 | E.O. | 50 F | Climacteric disturbance | stiff shoulder | III | " | 4 M |
| 24 | T.S. | 51 F | " | dizziness, headache | II | " | 4 Y |
| 25 | T.S. | 35 F | Vertigo | dizziness | III | " | 15 D |
| 26 | M.M. | 36 F | Autonomic imbalance | headache, insomnia | IV | " | 2 W |
| 27 | H.Y. | 47 F | " | insomnia | IV | " | 2 Y |
| 28 | S.W. | 42 F | " | nausea, vomiting | IV | " | 3 M |
| 29 | K.S. | 30 F | " | headache, vomiting | IV | " | 1 M |
| 30 | S.M. | 45 F | " | stiff shoulder, lumbago | IV | " | 2 M |

| Case report No. | 1 | | 2 | | 3 | | 4 | | 5 | | 6 | | 7 | | 8 | | 9 | | 10 | |
|---|---|---|---|---|---|---|---|---|---|---|---|---|---|---|---|---|---|---|---|---|
| Dosage (mg × times) | 2 × 6 | | 2 × 6 | | 2 × 10 | | 5 × 16 | | 5 × 10 | | 5 × 18 | | 2 × 6 | | 2 × 12 | | 2 × 10 | | 5 × 10 | |
| Period of administration | 3 W | | 3 W | | 5 W | | 7 W | | 8 W | | 12 W | | 5 W | | 6 W | | 6 W | | 10 W | |
| (Subjective symptoms) | | | | | | | | | | | | | | | | | | | | |
| B: Before administration A. After administration | B | A | B | A | B | A | B | A | B | A | B | A | B | A | B | A | B | A | B | A |
| Easily tired | +++ | — | +++ | — | +++ | ± | ++ | — | +++ | — | +++ | ++ | +++ | — | +++ | + | ++ | — | +++ | — |
| Feverish feeling | | | + | — | | | | | | | + | — | | | + | — | | | | |
| Sensitive to cold | | | | | | | | | + | — | ++ | — | | | | | ++ | — | | | + | — |
| Sweating | | | | | | | | | | | | | | | | | | | | |
| Emaciation | ++ | — | | | | | | | | | | | | | | | | | | |
| Insomnia | | | ++ | — | | | + | — | ++ | — | ++ | — | | | | | +++ | — | ++ | — |
| Vertigo | + | — | + | — | | | +++ | — | +++ | — | +++ | — | | | ++ | — | ++ | — | ++ | — |
| Ear noises | | | | | | | | | | | +++ | ++ | | | | | | | | |
| Headache, Dull head | | | | | + | — | ++ | — | | | ++ | — | | | ++ | — | +++ | — | +++ | — |
| Lumbago | | | | | | | | | | | + | — | ++ | + | + | — | ++ | — | | |
| Stiff shoulder | + | — | ++ | — | ++ | — | +++ | — | ++ | — | ++ | ± | +++ | + | +++ | — | +++ | — | +++ | — |

0 012 774

| Case report No. | 1 | 2 | 3 | 4 | 5 | 6 | 7 | 8 | 9 | 10 |
|---|---|---|---|---|---|---|---|---|---|---|
| Numbness of extremities | | | | | | | | | | |
| Tremor of finger and eyelid | | | | + + | | | | | | |
| Palpitation | | | | | ++ — | | | + — | ++ — | |
| Breathlessness | | | | | ++ — | | | ++ — | | |
| Oppression in chest | | | | | | | | | | |
| loss of appetite | +++ — | | | ++ — | | ++ — | | ++ — | +++ — | ++ — |
| Thirst | + — | | | | | | +++ — | ++ — | | |
| Nausea and vomiting | ++ — | | | | + — | | | | | +++ — |
| Abdominal disorder and dilation | ++ — | | | + — | | + + | + — | | ++ — | + — |
| Constipation and diarrhea | ++ — | | | | ++ — | | + — | | ++ — | ++ — |
| Abdominal pain | ++ — | | | ++ — | | | | | | + — |
| Asthenopia | ++ — | + — | ++ — | + — | ++ — | + — | | ++ — | | ++ — |
| EFFECT (E: Effective, V E: Very effective) | V E | V E | E | V E | V E | E | E to some degree | V E | V E | E |
| Adverse reaction | none | none | none | none | none | none | none | none | none | none |

| Case report No. | 11 B | 11 A | 12 B | 12 A | 13 B | 13 A | 14 B | 14 A | 15 B | 15 A | 16 B | 16 A | 17 B | 17 A | 18 B | 18 A | 19 B | 19 A | 20 B | 20 A |
|---|---|---|---|---|---|---|---|---|---|---|---|---|---|---|---|---|---|---|---|---|
| Dosage (mg × times) | 2 × 9 | | 5 × 5 | | 5 × 2 | | 2 × 6 | | 2 × 4 | | 5 × 2 | | 2 × 3 | | 5 × 4 | | 2 × 8 | | 2 × 8 | |
| Period of administration | 3 W | | 6 W | | 1 W | | 3 W | | 2 W | | 2 W | | 5 W | | 4 W | | 4 W | | 4 W | |
| (Subjective symptoms) | | | | | | | | | | | | | | | | | | | | |
| B: Before administration / A: After administration | B | A | B | A | B | A | B | A | B | A | B | A | B | A | B | A | B | A | B | A |
| Easily tired | +++ | + | +++ | ++ | ++ | — | +++ | ++ | ++ | — | +++ | +++ | +++ | — | +++ | — | ++ | — | +++ | — |
| Feverish feeling | | | + | — | | | ++ | — | | | | | | | | | | | | |
| Sensitiveness to cold | | | ++ | — | | | ++ | — | ++ | — | | | + | — | ++ | — | | | | |
| Sweating | | | + | — | | | ++ | — | | | | | | | | | | | | |
| Emaciation | | | | | | | | | | | | | | | | | ++ | — | | |
| Insomnia | ++ | — | | | | | | | | | | | ++ | — | ++ | — | | | +++ | — |
| Vertigo | ++ | — | ++ | — | +++ | — | ++ | — | | | | | +++ | + | +++ | — | | | | |
| Ear noises | | | | | | | | | ++ | ++ | | | | | | | | | +++ | — |
| Headache, Dull head | | | +++ | ++ | ++ | ++ | ++ | — | | | | | +++ | + | + | — | | | | |
| Lumbago | | | | | | | | | | | | | | | | | | | + | — |
| Stiff shoulder | ++ | — | ++ | + | ++ | — | +++ | + | ++ | — | | | ++ | — | +++ | — | ++ | — | ++ | + |

| Case report No. | 11 | 12 | 13 | 14 | 15 | 16 | 17 | 18 | 19 | 20 |
|---|---|---|---|---|---|---|---|---|---|---|
| Numbness of extremities | | + — | | + — | + — | +++ +++ | | + — | | |
| Tremor of finger and eyelid | | + — | | | | | | | | |
| Palpitation | | ++ — | | +++ — | ++ — | | | | | +++ — |
| Breathlessness | | + — | | +++ — | | | | ++ — | | |
| Oppression in chest | +++ — | + — | | ++ — | | | | | | |
| Loss of appetite | | ++ — | ++ — | + — | | | ++ — | +++ — | + — | +++ — |
| Thirst | | | | | | | ++ + | | | |
| Nausea and vomiting | ++ — | ++ — | | +++ — | | | +++ — | +++ — | | |
| Abdominal disorder and dilation | ++ — | + — | | + + | | +++ ++ | + — | ++ — | ++ ++ | |
| Constipation and diarrhea | | ++ — | | ++ + | | | | | | |
| Abdominal pain | | + — | | | | | | | ++ — | |
| Asthenopia | | ++ — | | + — | | | ++ — | ++ — | | ++ — |
| EFFECT (E: Effective, V E: Very effective) | V E | E | E | E | V E | Discontinued | E | V E | E | V E |
| Adverse reaction | none | none | none | none | none | none | none | none | none | none |

| Case report No. | 21 | | 22 | | 23 | | 24 | | 25 | | 26 | | 27 | | 28 | | 29 | | 30 | |
|---|---|---|---|---|---|---|---|---|---|---|---|---|---|---|---|---|---|---|---|---|
| Dosage (mg × times) | 2 × 10 | | 5 × 4 | | 5 × 4 | | 5 × 4 | | 2 × 4 | | 5 × 1 | | 2 × 4 | | 2 × 16 | | 2 × 18 | | 2 × 8 | |
| Period of administration | | | | | | | | | | | | | | | | | 9 W | | 6 W | |
| (Subjective symptoms) | | | | | | | | | | | | | | | | | | | | |
| B: Before administration A: After administration | B | A | B | A | B | A | B | A | B | A | B | A | B | A | B | A | B | A | B | A |
| Easily tired | +++ | — | +++ | — | +++ | — | +++ | ++ | +++ | +++ | +++ | — | +++ | — | +++ | — | +++ | — | +++ | — |
| Feverish feeling | ++ | — | + | — | | | ++ | — | | | | | | | | | | | | |
| Sensitiveness to cold | | | | | | | ++ | ++ | ++ | ++ | | | | | | | ++ | — | ++ | — |
| Sweating | | | | | | | ++ | — | | | ++ | — | | | | | | | | |
| Emaciation | | | | | | | | | | | | | ++ | — | | | | | | |
| Insomnia | + | — | | | | | | | | | +++ | — | +++ | — | | | ··· | · | ++ | — |
| Vertigo | | | | | | | +++ | — | +++ | + | ++ | — | ++ | — | | | +++ | — | | |
| Ear noises | | | | | | | | | | | | | | | | | | | | |
| Headache, Dull head | +++ | — | +++ | — | | | +++ | — | ++ | ++ | +++ | — | | | ++ | — | +++ | + | + | — |
| Lumbago | | | ++ | ++ | | | | | | | | | | | +++ | — | | | ++ | + |
| Stiff shoulder | + | — | +++ | — | +++ | — | +++ | ++ | | | ++ | — | | | +++ | — | +++ | — | +++ | — |

| Case report No. | 21 | 22 | 23 | 24 | 25 | 26 | 27 | 28 | 29 | 30 |
|---|---|---|---|---|---|---|---|---|---|---|
| Numbness of extremities | | ++ — | | + — | | + — | | | ++ — | |
| Tremor of finger and eyelid | | | | | | | | | | |
| Palpitation | | +++ — | | +++ — | ++ ++ | ++ — | | | | |
| Breathlessness | +++ — | | +++ — | | +++ — | | | | | |
| Oppression in chest | | | | +++ — | ++ + | +++ — | | | | |
| Loss of appetite | + — | | | | | ++ — | +++ — | | +++ — | ++ — |
| Thirst | | | | | | | | | | |
| Nausea and vomiting | | | | | | ++ — | ++ — | +++ — | +++ — | |
| Abdominal disorder and dilation | | | | | | ++ — | | | | |
| Constipation and diarrhea | | +++ — | | | ++ + | +++ — | ++ — | | | + — |
| Abdominal pain | | | ++ — | | | | | | | |
| Asthenopia | + — | | | ++ — | | | | +++ — | ++ — | ++ — |
| EFFECT (E: Effective, V E: Very effective) | V E | V E | E | E | No effect | V E | V E | V E | V E | V E |
| Adverse reaction | none | none | none | none | none | none | none | none | none | none |

Brief description of drawings

Figure 1 is an ultra-violet absorption spectrum of the extract of human umbilical cords of this invention; and Figure 2 is a chart of amino acid analysis which indicates the amino acid composition of the human umbilical cord extract of Figure 1.

The abbreviations used in Figure 2 are the following:

| | | |
|---|---|---|
| 1. Lysine | 2. Histidine | 3. Ammonia |
| 4. Arginine | 5. Aspartic acid | 6. Proline |
| 7. Serine | 8. Glutamic acid | 9. Proline |
| 10. Glycine | 11. Alanine | 13. Valine |
| 14. Methionine | 15. Isoleucine | 16. Leucine |
| 17. Tyrosine | 18. Phenylalanine | 12. Cysteine |

Best modes for carrying out the invention

The following paragraphs illustrate manufacture of autonomic imbalance treating agents according to the invention.

1A. Umbilical cords were separated from healthy human placentae and blood was removed by making small cuts on the blood vessels. The cords were then washed thoroughly with cold water, the water allowed to drain thoroughly and the cords were frozen for 2—3 days. The frozen umbilical cords were allowed to stand at room temperature and when half thawed, chopped with a chopper into small pieces. Then to the chopped umbilical cords 5 times as much water as the umbilical cords by weight and purified proteolytic enzyme obtained from *Streptomyces griceus* were added and incubated at 45—50°C for 10—16 hours to effect decomposition of constituents of the cords. Then the resulting liquid was heated at 120°C for 10—15 minutes and thereafter cooled and filtered.

1B. To the filtrate, 0.71% of sodium chloride and 0.3% of benzyl alcohol were added and dissolved, this solution was filtered through a microporous filter (e.g. Millipore filter — the word "Millipore" is a Trade Mark) to remove microorganisms. The filtrate was filled into ampoules, dry heat sterilized, and then sterilized in an autoclave at 120°C for 10 minutes and used for injection.

2. To the enzyme-decomposed liquid obtained by the same process as in paragraph 1A, 10% citric acid solution was added to adjust it to pH 4.6 and it was stirred gently for 5—10 minutes at 45—50°C and then cooled and filtered. To the filtrate, 0.05% by weight active carbon was added and mixed well and filtered. The filtrate was adjusted to pH 6.5 with 10% sodium carbonate solution and then 0.5% of chlorobutanol and 0.7% of sodium chloride were added and dissolved, then the filtrate was filtered through a Millipore filter to remove microorganisms. The filtrate was filled into dry heat sterilized ampoules in aseptic conditions and used for injection.

3. To the enzyme-decomposed liquid obtained by the same process as in paragraph 1A, 10% hydrochloric acid was added to adjust it to pH 4.6 and the liquid was kept at 45—50°C for 5—10 minutes and gently stirred. Then the liquid was cooled and filtered, and to the filtrate, 0.05% of active carbon was added and mixed and the liquid was filtered. The filtrate was adjusted to pH 6.5 by adding 10% sodium carbonate and to this, 0.7% of sodium chloride and 0.3% of benzylalcohol were added and filtered by a Millipore filter to remove microorganisms. The aseptic filtrate was filled into dry heat sterilized ampoules and sterilized in an autoclave for 10 minutes at 120°C and used for injection.

## Claims

1. An autonomic imbalance treating agent prepared by treating comminuted human umbilical cords in an aqueous medium with a proteolytic enzyme to effect enzymic decomposition, removing proteins from the resulting solution and sterilizing the same free of proteins.

2. An autonomic imbalance treating agent as claimed in claim 1, wherein said proteolytic enzyme is selected from proteolytic enzymes of animal origin, proteolytic enzymes of microorganic origin and proteolytic enzymes of vegetable origin.

3. An autonomic imbalance treating agent as claimed in claim 2, wherein the proteolytic enzymes of animal origin are selected from pepsin, trypsin and chymotrypsin.

4. An autonomic imbalance treating agent as claimed in claim 2, wherein the proteolytic enzymes of microorganic origin are derived from *Streptomyces griseus, Bacillus subtilis* or other *Bacillus* species, *Aspergillus Melius, Aspergillus niger* or *Serratia*.

5. An autonomic imbalance treating agent according to claim 2, wherein the proteolytic enzyme of vegetable origin is bromelain, papain or ficin.

6. An autonomic imbalance treating agent as claimed in any of claims 2 to 5, wherein said proteolytic enzyme is used in the form of a fixed enzyme.

7. An autonomic imbalance treating agent as claimed in any preceding claim, wherein said protein-removing treatment comprises either heating said resulting solution or adjusting the pH thereof to 4—5.

21

**Revendications**

1. Agent thérapeutique pour le traitement de déséquilibres vasomoteurs, préparé par traitement de cordons ombilicals broyés avec une enzyme protéolitique dans un milieu aqueux pour effectuer une décomposition enzymique, éloignement des protéines de la solution résultante et stérilisation de cette solution libre de protéines.

2. Agent thérapeutique pour le traitement de déséquilibres vasomoteurs selon la revendication 1, caractérisé en ce que l'enzyme protéolytique est choisi parmi les enzymes protéolitiques d'origine animale, les enzymes protéolytiques d'origine microorganique et les enzymes protéolytiques d'origine végétale.

3. Agent thérapeutique pour le traitement de déséquilibres vasomoteurs selon la revendication 2, caractérisé en ce que les enzymes protéolytiques d'origine animale sont choisi parmi la pepsine, la trypsine et la chymotrypsine.

4. Agent thérapeutique pour le traitement de déséquilibres vasomoteurs selon la revendication 2, caractérisé en ce que les enzymes protéolytiques d'origine microorganique sont dérivés de streptomyces griseus, bacillus subtilis ou autres espèces de bacillus, aspergillus melius, aspergillus niger et serratia.

5. Agent thérapeutique pour le traitement de déséquilibres vasomoteurs selon la revendication 2, caractérisé en ce que l'enzyme protéolytique d'origine végétale est la bromélaine, la papaine ou la ficine.

6. Agent thérapeutique pour le traitement de déséquilibres vasomoteurs selon l'une des revendications 2 à 5, caractérisé en ce que l'enzyme protéolytique est employée sous forme d'une enzyme fixée.

7. Agent thérapeutique pour le traitement de déséquilibres vasomoteurs selon l'une des revendications précédentes, caractérisé en ce que le traitement pour l'éloignement des protéines comprend soit le chauffage de la solution résultante ou ajustement du pH de celle-ci à 4—5.

**Patentansprüche**

1. Mittel zur Behandlung vasomotorischer Störungen, hergestellt durch Behandlung zerkleinerter menschlicher Nabelschnüre in einem wässrigen Medium mit einem proteolytischen Enzym zur Siegelung einer enzymatischen Zersetzung, Entfernung der Proteine aus der erhaltenen Lösung und Sterilisierung der proteinfreien Lösung.

2. Mittel zur Behandlung vasomotorischer Störungen, nach Anspruch 1, dadurch gekennzeichnet, dass das proteolytische System ausgewählt ist aus proteolytischen Enzymen tierischen Ursprungs, proteolytischen Enzymen mikroorganischen Ursprungs und proteolytischen Enzymen pflanzlichen Ursprungs.

3. Mittel zur Behandlung vasomotorischer Störungen, nach Anspruch 2, dadurch gekennzeichnet, dass die proteolytischen Enzyme tierischen Ursprungs ausgewählt sind aus Pepsin, Trypsin und Chymotrypsin.

4. Mittel zur Behandlung vasomotorischer Störungen nach Anspruch 2, dadurch gekennzeichnet, dass die proteolytischen Enzyme mikroorganischen Ursprungs abgeleitet sind von Streptomyces griseus, Bacillus suptilis oder anderen Bacillusarten, Aspergillus Melius, Aspergillus niger oder Sarratia.

5. Mittel zur Behandlung vasomotorischer Störungen nach Anspruch 2, dadurch gekennzeichnet, dass das proteolytische System pflanzlichen Ursprungs Bromelain, Papain oder Ficin ist.

6. Mittel zur Behandlung vasomotorischer Störungen nach einem der Ansprüche 1 bis 5, dadurch gekennzeichnet, dass das proteolytische Enzym in Form eines fixierten Enzyms verwendet wird.

7. Mittel zur Behandlung vasomotorischer Störungen nach einem der vorstehenden Ansprüche, dadurch gekennzeichnet, dass die protein-entfernende Behandlung entweder die Erhitzung der erhaltenen Lösung oder das Einstellen ihres pH-Wertes auf 4—5 umfasst.

# FIG.1

FIG. 2